# EUROPEAN PATENT APPLICATION

(11) **EP 2 764 860 A1**
(43) Date of publication of application: **13.08.2014**
(21) Application number: 13154207.8
(22) Date of filing: 06.02.2013
(51) Int. Cl.: A61K 8/44, A61K 8/92, C11B 1/04

(54) **Cupuassu fatty acid amidoamines and their derivatives**

(71) Applicant: BASF SA, 04538-132 Sao Paulo (BR)
(72) Inventor: Sanchez, Agustin, 04552-050 Sao Paulo (BR); Sales, Henrique, 04552-050 Sao José dos Campos (SP) (BR); Oliveira, Mariele, 04552-050 Sao José dos Campos (SP) (BR); Leite, Eliane, 04552-050 Sao José dos Campos (SP) (BR); Sato, Anne, 04552-050 Sao José dos Campos (SP) (BR); Pereira, Diana Estefani Ferreira Eugenio, 04552-050 Jacarei (SP) (BR)
(74) Representative: Reinhardt, Jürgen

(57) **Abstract**

Suggested is a cupuassu fatty acid amidoamine according to formula (I) 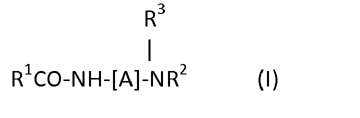 in which
R¹CO
stands for a mixture of acyl radicals having 12 to 22 carbon atoms;
R² and R³
independently from each other represent hydrogen or alkyl radicals having 1 to 6 carbon atoms;
A
stands for a linear or branched alkylene radical having 1 to 6 carbon atoms
on condition that the mixture of acyl radicals represent the fatty acid spectrum of cupuassu butter (*Theobroma grandiflorum*). The invention also encompasses the respective quaternization products and the mixtures of said quaternized and said non-quaternized amidoamines.

## Description

### Field of invention

The present invention belongs to the area of personal care and cosmetic compositions and refers to new fatty acid amidoamines and their quaternized derivatives based on cupuassu butter, a process for obtaining these compounds and various compositions comprising them.

### State of the art

Nitrogen containing materials - either being surfactants or polymers - have been found to be particularly effective for controlling viscosity, providing lubrication during rinse off, reducing wet and dry combing force, improving shine, improving the texture and the shape of the hair and make the skin feel pleasant to touch. Although certain cationic materials are known to provide such benefits, there is an on-going need for alternatives with improved performance.

A particular useful class of cationic or pseudo-cationic surfactants encompass derivatives of fatty acid amides, especially fatty acid amidoamines, that can be obtained from the reaction of fatty acids and di- or polyamines that contain one tertiary amine group, optionally followed by a quaternization using suitable alkylation agents. Amidoamines and their quaternization products are known from the prior art. For example, European patent EP 0465734 B1 (Helene Curtis) discloses fatty acid amidoamines for hair care preparations. European patent EP 0859587 B1 (P&G) refers to hair conditioners also comprising fatty acid amidoamines. Personal care compositions comprising stearamidopropyl dimethylamine and behenamidopropyl dimethylamine are disclosed in International patent application WO 2007 120681 A2 (Inolex).

Nevertheless, these compounds are still not satisfying with regard to their performance on human skin and hair. Therefore, it has been the object of the present invention to develop new amidoamines and their quaternization products exhibiting simultaneously superior qualities with regard to conditioning, moisturizing, and hair gloss, removal of static charge, splin-end removal and tangling of wet hair.

### Description of the invention

A first object of the present invention is a cupuassu fatty acid amidoamine according to formula (I) 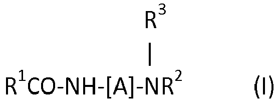 in which
R¹CO stands for a mixture of acyl radicals having 12 to 22 carbon atoms;
R² and R³ independently from each other represent hydrogen or alkyl radicals having 1 to 6 carbon atoms;
A stands for a linear or branched alkylene radical having 1 to 6 carbon atoms on condition that the mixture of acyl radicals represent the fatty acid spectrum of cupuassu butter *(Theobroma grandiflorum).*

A second object of the present invention relates to a quaternary cupuassu fatty acid amidoamine according to formula (II) 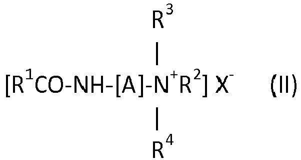 in which
R¹CO stands for a mixture of acyl radicals having 12 to 22 carbon atoms;
R², R³ and R⁴ independently from each other represent hydrogen or alkyl radicals having 1 to 6 carbon atoms;
A stands for a linear or branched alkylene radical having 1 to 6 carbon atoms; and
X represents a halogenide, sulfate or carbonate anion;
on condition that the mixture of acyl radicals represent the fatty acid spectrum of cupuassu butter *(Theobroma grandiflorum).*

A third object of the present invention is directed to a mixture of a cupuassu fatty acid amidoamines of formula (I) and a of formula (II) or a quaternary conditioning agent in a weight ratio of about 10:90 to about 90:10

Surprisingly, it has been observed that cupuassu fatty acid amidoamines and their mixtures with alone or in particular in combination exhibit superior performance with respect to the treatment of human hair and skin when compared with conventional amidoamines or quaternaries that are obtained from different renewable sources. In particular it has been found that the products
- better improve moisturizing of skin and hair;
- better improve combability of human hair by reducing the electric charge;
- provide superior conditioning and gloss to human hair.

### Cupuassu fatty acid amidoamines and their quaternized derivatives

As explained above, amidoamines of formula (I) and their quaternization products according to formula (II) are basically known from the prior art. These products are obtained either from discrete fatty acids or fatty acid fractions obtained for example from palm oil or coconut oil. However, the particular advantage of the present invention over the cited prior art is the fact that the species are derived from the oil of *theobroma grandiflorum,* also called cupuassu butter, that exhibits are very special fatty acid profile that is significantly different from the plant sources commonly used.

Cupuassu, also spelled cupuaçu, cupuazú, or copoasu, is a tropical rainforest tree related to cacao. Common throughout the Amazon basin, it is widely cultivated in the jungles of Colombia, Bolivia and Peru and in the north of Brazil, with the largest production in Pará, followed by Amazonas, Rondônia and Acre. Cupuassu butter contains a high amount of phytochemicals, such as polyphenolic tannins, theograndins I and II, and flavonoids, including catechins, quercetin, kaempferol and isoscutellarein. The fatty acid spectrum comprises mainly C14, C16, C18 and C20 fatty acids, with high amounts of stearic and arachidonic acid.

The preparation of Cupuassu butter is described, for instance, in European patent application EP 1219698 A1**;** see especially the examples on pages 7 to 9 of this application, which is hereby incorporated by reference. In this context reference is also made to European patent EP 1786394 B1 (Cognis Brasil) that is related to amphoteric surfactants based on cupuassu butter.

As also explained above, both the amidoamines and their quaternization products are useful for solving the technical problem underlying the present invention, however, a synergistic effect can be observed when using mixtures of both components. Such mixtures can be obtained by blending the species; however in a preferred embodiment of the present invention such mixtures are obtained *in-situ* in that cupuassu butter is subjected to transamidation and the amidoamines thus obtained are partly subjected to quaternization, whereby the amount of quaternization agent is adjusted so that the desired ratio of non-quaternized and quaternized amidoamines is automatically achieved.

Synergistic mixtures comprise the amidoamines and their quaternization products in weight ratios of about 10:90 to about 90:10, preferably about 25:75 to about 75:25 and more preferably about 40:60 to about 60:40.

In a preferred embodiment of the invention, the mixtures of amidoamines and their quaternization products may comprise phytochemicals, particularly phytosterols such as described above in amounts up to 5 % b.w. and preferably within the range of about 0.1 to 2 % .w.

### Transamidation and Quaternization

Another object of the present invention is directed to a process for making cupuassu fatty acid amidoamines and/or quaternized cupuassu fatty acid amidoamines in that
(a) the oil from *Theobroma grandiflorum* is subjected to transamidation with a diamine of formula (III)

   NH₂-[A]-NR²R³ (III)

   wherein A, R² and R³ have the same meanings as defined above, and optionally
(b) the cupuassu fatty acid amidoamine thus obtained is reacted with an alkyl halogenide, a dialkyl sulfate or a dialkyl carbonate to provide the quaternized cupuassu fatty acid amidoamine.
Instead of the oil, also partial glycerides or alkyl esters, preferably methyl esters can be used.

Suitable amines according to formula (III) for carrying out the transamidation of the oils are selected from the group consisting of etehylenediamine, propylenediamine, butylenediamine, dimethylaminoethylamine, dimethylaminopropylamine, dimethylaminobutylamine, dimethylaminopentylamine, dimethylaminohexylamine, diethylamineethylamine, diethylaminopropylamine, diethylaminobutylamine, diethylaminopentylamine, diethylaminohexylamine, methylethylaminoethylamine, methylethylaminopropylamine, methylethylaminobutylamine, methylethylaminopentylamine, methylethylaminohexylamine and their mixtures. Preferred diamines are dimethylethylamine and dimethylpropylamine.

Transamidation of fatty acid alkyl esters or the respective glycerides is well known from the state of the art and disclosed for example in DE 4340423 C1**,** DE 4207386 C1 or DE 4030084 C1 (all Henkel). Typically, the reaction between the ester and the diamine takes place in the presence of an acidic catalyst. Hypophosphoric acid has been shown to be very effective, since it also has reducing effects that improves the colour of the reaction products. The reaction is typically carried out in a stirred vessel and at temperatures from about 70 to about 100 °C. Samples are taken regularly and once the mixture has reached an acid value of less than 5 - which requires typically about 2 to 3 hours - the products are cooled to room temperature. The amount of diamines is typically chosen so that it is sufficient to fully replace all alcohol groups in the ester. Typically, a small excess of up to 5 % is used. If necessary, unreacted diamine is removed from the reaction mixture by distillation.

Typically, the amidoamines are neutralized by adjusting the pH-value using for example lactic or citric acid. Therefore, it goes without saying that formula (I) also encompasses the neutralized species. Preferred species also encompass a neutralized cupuassu fatty acid amidoamine according to formula (Ib)ok 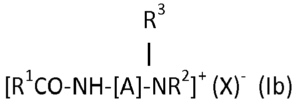 in which
R¹CO stands for a mixture of acyl radicals having 12 to 22 carbon atoms;
R² and R³ independently from each other represent hydrogen or alkyl radicals having 1 to 6 carbon atoms;
A stands for a linear or branched alkylene radical having 1 to 6 carbon atoms, and
X stands for a lactate or citrate anion,
on condition that the mixture of acyl radicals represent the fatty acid spectrum of cupuassu butter *(Theobroma grandiflorum).*

In case quaternized products or in-situ mixtures of quaternized and non-quaternized amidoamines are desired the products thus obtained are subjected to quaternization, fully or in part. Suitable quaternization agents encompass for methyl chloride, butyl chloride, dimethyl sulfate or dimethyl carbonate. Also quaternization represents a chemical operation that is well known from the state of the art. Typically, the alkylation agent is added drop wise to a solution of the amidoamine in a suitable solvent, such as for example isopropyl alcohol or ethylene glycol. It is desirous to use a solvent that is not needed to be removed from the reaction mixture, since it can also serve as a valuable component of the final composition. Typical examples encompass fatty alcohols and particularly fatty alcohol polyglycolethers. Since quaternization is a very exothermic reaction temperature is typically kept in a range of about 40 to about 60 °C, if necessary using an ice bath. Traces of unreacted alkylation agents in the final compositions are absolutely unwanted. Therefore, quaternization is typically carried out with a sub-stoichiometric amount of the alkylation agent which is typically about 2 to 5 Mol-%. Using the alkylation agents in amounts of about 95 to 98 Mol-% calculated on the molar amount of the alkanolamines leads to almost complete conversion and provides the quaternized amidoamine in yields of about 100 % of the theory.

In case it is intended to prepare in-situ mixtures of quaternized and non-quaternized amidoamines the fatty acid amidoamine intermediate and the quaternization agent are reacted in a molar ratio so that the resulting mixture comprises the non-quaternized and the quaternized fatty acid amidoamine in any desired weight ratio of about 10:90 to about 90:10. For example, in order to achieve a 1:1 mixture the amount of alkylation agent is reduced to about 50 %, which is sufficient to convert not more than 50 % of the amidoamines into their quaternization products.

### Industrial application

Additional objects of the present invention are directed to
(a) a personal care composition,
(b) a cosmetic composition or a
(c) a pharmaceutical composition
comprising
(a) a cupuassu fatty acid amidoamine, and
(b1) a quaternized cupuassu fatty acid amidoamine and/or
(b2) a quaternary conditioning agent.

Each of these compositions may comprise the cupuassu fatty acid amidoamines and/or the quaternized fatty acid amidoamines in a total amount of from about 0.5 to about 25 % b.w., preferably about 1 to about 20 % by weight, and more preferably about 2 to about 15 % b.w. - calculated on the final compositions. The ratio by weight between component (a) and components (b1+b2) can be 90:10 to 10:90, preferably 75:25 to 25:75 and even more preferred 60:40 to 40:60. Within the preferred ranges a strong synergistic behaviour is obtained, either taking the quaternized fatty acid amidoamines or the quaternary conditioning agents alone or a combination of both. ok

The compositions may also include lactic acid in an amount of from about 0.5 to about 25 % b.w., and more preferably of from about 1 to about 15 % b.w. ok

### Quaternary conditioning agents

Quaternary conditioning agents forming component (b2) may encompass monomeric and/or polymeric structures. Among the monomers cationic surfactants as for example tetra alkyl ammonium salts and preferably esterquats form the most prominent examples.

### Esterquats

"Esterquats" are generally understood to be quaternised fatty acid triethanolamine ester salts. These are known compounds which can be obtained by the relevant methods of preparative organic chemistry. Reference is made in this connection to International patent application WO 91/01295 (Henkel), according to which triethanolamine is partly esterified with fatty acids in the presence of hypophosphorous acid, air is passed through the reaction mixture and the whole is then quaternised with dimethyl sulphate or ethylene oxide. In addition, German patent DE 4308794 C1 (Henkel) describes a process for the production of solid esterquats in which the quaternisation of triethanolamine esters is carried out in the presence of suitable dispersants, preferably fatty alcohols. Overviews on this theme have been published by R. Puchta et al. in Tens. Surf. Det., 30, 186 (1993**),** by M. Brock in Tens. Surf. Det., 30, 394 (1993**),** by R. Lagerman et al. in J. Am. Oil Chem. Soc., 71, 97 (1994**)** and by I. Shapiro in Cosm. Toil. 109, 77 (1994**).** Typical examples of esterquats suitable for use in accordance with the invention are products of which the acyl component derives from monocarboxylic acids corresponding to formula (IV):

R¹CO-OH (IV)

in which R¹CO is an acyl group containing 6 to 10 carbon atoms. Examples of such monocarboxylic acids are caproic acid, caprylic acid, capric acid and technical mixtures thereof such as, for example, so-called head-fractionated fatty acid. Esterquats, of which the acyl component derives from monocarboxylic acids of formula (IV), in which R¹CO is a linear, saturated acyl group containing 8 to 10 carbon atoms, are preferably used.

Other esterquats used in accordance with the invention are those of which the acyl component derives from dicarboxylic acids corresponding to formula (V):

HOOC(CH₂)ₙCOOH (V)

in which n is a number of 1 to 10. Examples of such dicarboxylic acids are malonic acid, succinic acid, maleic acid, fumaric acid, glutaric acid, sorbic acid, pimelic acid, azelaic acid, sebacic acid and/or dodecanedioic acid, but preferably adipic acid. Overall, esterquats of which the acyl component derives from a mixture of (a) monocarboxylic acids corresponding to formula (I), where R¹CO is a linear saturated acyl group containing 6 to 22 carbon atoms, and of (b) adipic acid are preferably used. The molar ratio of acyl moiety (a) to acyl moiety (b) may be in the range from 1:99 to 99:1 and is preferably in the range from 50:50 to 90:10 and more particularly in the range from 70:30 to 80:20.

A special embodiment of the invention is characterized by the use of esterquats which represent quaternised fatty acid triethanolamine ester salts corresponding to formula (VI):

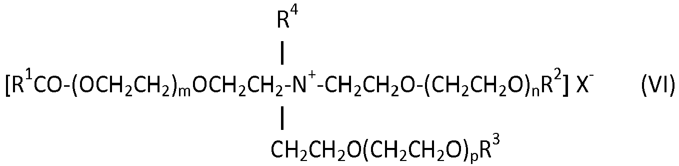

in which R¹CO stands for mono- and/or dicarboxylic acids, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulphate or alkyl phosphate.

Besides the quaternised fatty acid triethanolamine ester salts, other suitable esterquats are quaternised ester salts of mono- and/or dicarboxylic acids with diethanol alkylamines corresponding to formula (VII):

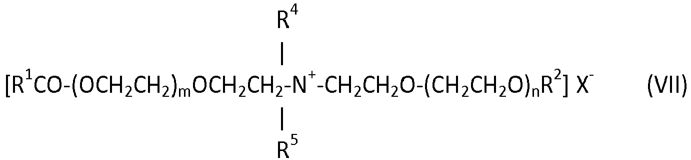

in which R¹CO represents a mono- and/or dicarboxylic acids R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulphate or alkyl phosphate.

Another group of suitable esterquats are the quaternised ester salts of mono- and/or dicarboxylic acid mixtures with 1,2-dihydroxypropyl dialkyl amines corresponding to formula (VIII): 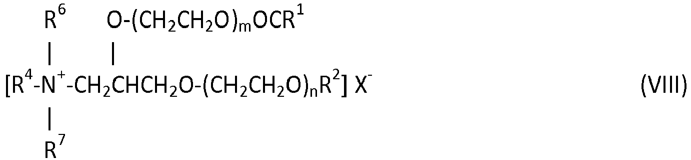 in which R¹CO represents a mono- and/or dicarboxylic acids, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulphate or alkyl phosphate.

In addition, other suitable esterquats are substances in which the ester bond is replaced by an amide bond and which - preferably based on diethylene triamine - correspond to formula (IX):

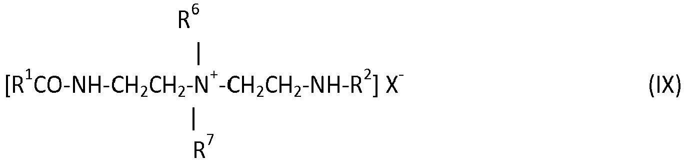

in which R¹CO represents a mono- and/or dicarboxylic acids, R² is hydrogen or has the same meaning as R¹CO, R⁶ and R⁷ independently of one another are alkyl groups containing 1 to 4 carbon atoms and X is halide, alkyl sulphate or alkyl phosphate. Amide esterquats such as these are commercially obtainable, for example, under the name of Incroquat^{®}. (Croda).

Finally, other suitable esterquats are compounds based on ethoxylated castor oil or hydrogenation products thereof which preferably correspond to formula (X):

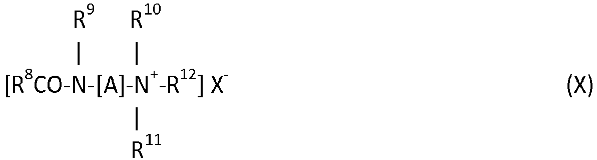

in which R⁸CO represents a mono- and/or dicarboxylic acids, A is a linear or branched alkylene group containing 1 to 6 carbon atoms, R⁹, R¹⁰ and R¹¹ independently of one another represent hydrogen or a C₁₋₄ alkyl group, R¹² is a C₁₋₄ alkyl group or a benzyl group and X is halogen, alkyl sulphate or alkyl phosphate. Such products are commercial available, for example, under the name of Demelan^{®} AU 39 BIO (Cognis)

So far as the choice of the preferred fatty acids and the optimal degree of esterification are concerned, the examples mentioned for (IV) also apply to the esterquats corresponding to formulae (V) to (X).

The esterquats corresponding to formulae (V) to (X) may be obtained both from fatty acids and from the corresponding triglycerides in admixture with the corresponding dicarboxylic acids. One such process, which is intended to be representative of the relevant prior art, is proposed in European patent EP 0750606 B1 (Cognis). To produce the quaternised esters, the mixtures of mono- and dicarboxylic acids and the triethanolamine--based on the available carboxyl functions--may be used in a molar ratio of 1.1:1 to 3:1. With the performance properties of the esterquats in mind, a ratio of 1.2:1 to 2.2:1 and preferably 1.5:1 to 1.9:1 has proved to be particularly advantageous. The preferred esterquats are technical mixtures of mono-, di- and triesters with an average degree of esterification of 1.5 to 1.9. Particularly preferred are esterquats and esterquats compositions derived from fatty acid triethanolamine esters like Dehyquart^{®} L80, Dehyquart^{®} F 75, Dehyquart^{®} F100 or Dehyquart^{®} C 4046 which are described in more detail in EP 1119658 B1**,** EP 1128808 B1**,** EP 1131049 B1 and WO 00/027344 A1 (BASF Personal Care & Nutrition GmbH); as far as these products are concerned the teaching of the cited references is herewith fully incorporated by reference.

### B. Cationic polymers

Suitable cationic polymers also suitable as conditioning agents are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethylene-imine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®} CBS, Jaguar^{®} C-17, Jaguar^{®} C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

### Methods of treatment

The invention also encompasses
(a) a first method for improving wettability, for providing moisture and for reducing electrostatic charge of human hair, and
(b) a second method for improving the moisture status of human skin, the improvement comprising appliying
   (i) the cupuassu fatty acid amidoamines,
   (ii) the quaternized cupuassu fatty acid amidoamines,
   (iii) their mixtures, or
   (iv) on of the compositions comprising quaternary conditioning agents
to hair or skin. ok

Finally, another object of the present invention is related to the use of cupuassu fatty acid amidoamines of formula (I), of quaternized cupuassu fatty acid amidoamines of formula (II) or their for making personal care, cosmetic or pharmaceutical compositions.

### Personal care, cosmetic and pharmaceutical compositions

The preparations according to the invention may contain surfactants, oil bodies, emulsifiers, superfatting agents, pearlising waxes, consistency factors, polymers, silicone compounds, waxes, stabilizers, primary and secondary sun protection agents, antidandruff agents, biogenic agents, film formers, swelling agents, hydrotropes, preservatives, solubilizers, complexing agents, reducing agents, alkalising agents, perfume oils, dyes and the like as additional auxiliaries and additives.

### A. Surfactants

Other preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oli-goglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### B. Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhy-droxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### C. Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
(i) **Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.
(ii) **Sorbitan esters.** Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.
(iii) **Polyglycerol esters.** Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.
(iv) **Anionic emulsifiers.** Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.
(v) **Amphoteric emulsifiers.** Other suitable emulsifiers are amphboteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### D. Superfatting agents and consistency factors

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### E. Thickening agents and rheology additives

Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### F. Polymers

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### G. Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### H. Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### I. Waxes and stabilizers

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

### J. Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic compositions, preferably topical formulations according to the present invention comprise one, two, three or more sun protection factors selected from the group consistiung of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivativesand indole derivatives.

In addition, it is advantageous to combine compounds of formula (I) with active ingredients which penetrate into the skin and protect the skin cells from inside against sunlight-induced damage and reduce the level of cutaneous matrix metalloproteases. Preferred respective ingredients, so called arylhydrocarbon receptor antagonists, are described in WO 2007/128723, incorporated herein by reference. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan^{®}HMS)
- salicylic acid-2-ethylhexyl ester (Neo Heliopan^{®}OS)
- triethanolamine salicylate
- 4-isopropyl benzyl salicylate
- anthranilic acid menthyl ester (Neo Heliopan^{®}MA)
- diisopropyl cinnamic acid ethyl ester
- p-methoxycinnamic acid-2-ethylhexyl ester (Neo Heliopan^{®}AV)
- diisopropyl cinnamic acid methyl ester
- p-methoxycinnamic acid isoamyl ester (Neo Heliopan^{®}E 1000)
- p-methoxycinnamic acid diethanolamine salt
- p-methoxycinnamic acid isopropyl ester
- 2-phenylbenzimidazole sulfonic acid and salts (Neo Heliopan^{®}Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan^{®}MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb^{®}HEB)
- benzylidene malonate polysiloxane (Parsol^{®}SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul^{®}T150)

Broadband filters which are preferably combined with one or more compounds of formula (I) in a preparation according to the present invention are selected from the group consisting of
- 2-ethylhexyl-2-cyano-3,3-diphenyl acrylate (Neo Heliopan^{®}303)
- ethyl-2-cyano-3,3'-diphenyl acrylate
- 2-hydroxy-4-methoxybenzophenone (Neo Heliopan^{®}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl®XL)
- 2,2'-methylene bis-(6-(2H-benzotriazol-2-yl)-4-1,1,3,3-tetramethylbutyl) phenol) (Tinosorb^{®}M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb^{®}S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine

UV-A filters filters which are preferably combined with one or more compounds of formula (I) in a preparation according to the present invention are selected from the group consisting of
- 4-isopropyl dibenzoyl methane
- terephthalylidene dibornane sulfonic acid and salts (Mexoryl^{®}SX)
- 4-t-butyl-4'-methoxydibenzoyl methane (avobenzone) / (Neo Heliopan^{®}357)
- phenylene bis-benzimidazyl tetrasulfonic acid disodium salt (Neo Heliopan^{®}AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul^{®} A Plus)
- indanylidene compounds in accordance with DE 100 55 940 A1 (= WO 2002 038537 A1)

UV filters which are more preferably combined with one or more compounds of formula (I) in a preparation according to the present invention are selected from the group consisting of
- p-aminobenzoic acid
- 3-(4'-trimethylammonium) benzylidene bornan-2-one methyl sulfate
- salicylic acid homomenthyl ester (Neo Heliopan^{®}HMS)
- 2-hydroxy-4-methoxybenzophenone (Neo Heliopan^{®}BB)
- 2-phenylbenzimidazole sulfonic acid (Neo Heliopan^{®}Hydro)
- terephthalylidene dibornane sulfonic acid and salts (Mexoryl^{®}SX)
- 4-tert-butyl-4'-methoxydibenzoyl methane (Neo Heliopan^{®}357)
- 3-(4'-sulfo)benzylidene bornan-2-one and salts
- 2-ethylhexyl-2-cyano-3,3-diphenyl acrylate (Neo Heliopan^{®}303)
- N-[(2 and 4)-[2-(oxoborn-3-ylidene) methyl]benzyl] acrylamide polymer
- p-methoxycinnamic acid-2-ethylhexyl ester (Neo Heliopan^{®}AV)
- p-aminobenzoic acid ethyl ester (25 mol) ethoxylated (INCI name: PEG-25 PABA)
- p-methoxycinnamic acid isoamyl ester (Neo Heliopan^{®}E1000)
- 2,4,6-trianilino-(p-carbo-2'-ethylhexyl-1'-oxy)-1,3,5-triazine (Uvinul^{®}T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan^{®}MBC)
- 3-benzylidene camphor
- salicylic acid-2-ethylhexyl ester (Neo Heliopan^{®}OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb^{®}M)
- phenylene bis-benzimidazyl tetrasulfonic acid disodium salt (Neo Heliopan^{®}AP)
- 2,4-bis-[{(4-(2-ethylhexyloxy)-2-hydroxy}phenyl]-6-(4-methoxyphenyl)-1,3,5-triazine (Tinosorb^{®}S)
- benzylidene malonate polysiloxane (Parsol^{®}SLX)
- menthyl anthranilate (Neo Heliopan^{®}MA)
- 2-(4-diethylamino-2-hydroxybenzoyl) benzoic acid hexyl ester (Uvinul^{®} A Plus)
- indanylidene compounds in accordance with DE 100 55 940 (= WO 02/38537).

Advantageous primary and also secondary sun protection factors are mentioned in WO 2005 123101 A1. Advantageously, these preparations contain at least one UVA filter and/or at least one UVB filter and/or at least one inorganic pigment. The preparations may be present here in various forms such as are conventionally used for sun protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

In a further preferred embodiment a formulation according to the invention contains a total amount of sunscreen agents, i.e. in particular UV filters and/or inorganic pigments (UV filtering pigments) so that the formulation according to the invention has a light protection factor of greater than or equal to 2 (preferably greater than or equal to 5). Such formulations according to the invention are particularly suitable for protecting the skin and hair.

### H. Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1**.** The total quantity of inorganic pigments, in particular hydrophobic inorganic micro-pigments in the finished cosmetic preparation according to the present invention is advantageously from 0.1 to 30% by weight, preferably 0.5 to 10.0% by weight, in each case based on the total weight of the preparation.

Also preferred are particulate UV filters or inorganic pigments, which can optionally be hydrophobed, can be used, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### J. Anti-ageing actives

In the context of the invention, anti-ageing or biogenic agents are, for example antioxidants, matrix-metalloproteinase inhibitors (MMPI), skin moisturizing agents, glycosaminglycan stimulators, anti-inflammatory agents, TRPV1 antagonists and plant extracts.
(i) **Antioxidants.** amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysylthreonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to µmol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnSO₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone.
(ii) **Matrix-Metalloproteinase inhibitors (MMPI).** Preferred compositions comprise matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonyl-fluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, which are listed in WO 02 069992 A1 (see tables 1-12 there, incorporated herein by reference), proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005 123101 A1**,** incorporated herein by reference) as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.
(III) **Skin-moisturizing agents.** Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.
(iv) **Glycosaminoglycan stimulators.** Preferred compositions comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCl: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCl: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.
(v) **Anti-inflammatory agents.** The compositions may also contain anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004 047833 A1**,** are preferred anti-itch ingredients in a composition according to the present invention. Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1**),** boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3-1,4-β-glucan from oats.
(vi) **TRPV1 antagonists.** Suitable compounds which reduce the hypersensitivity of skin nerves based on their action as TRPV1 antagonists, encompass e.g. trans-4-tert-butyl cyclohexanol as described in WO 2009 087242 A1**,** or indirect modulators of TRPV1 by an activation of the µ-receptor, e.g. acetyl tetrapeptide-15, are preferred.
(vii) **Botanical extracts.** The compositions may also contain various extracts of plants, such as for example extracts of *Ginkgo biloba, Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Melissa officinalis, Valeriana officinalis, Castanea sativa, Salix alba* and *Hapagophytum procumbens.*

### K. Cooling agents

The compositions may also contain one or more substances with a physiological cooling effect (cooling agents), which are preferably selected here from the following list: menthol and menthol derivatives (for example L-menthol, D-menthol, racemic menthol, isomenthol, neoisomenthol, neomenthol) menthylethers (for example (I-menthoxy)-1,2-propandiol, (I-menthoxy)-2-methyl-1,2-propandiol, I-menthyl-methylether), menthylesters (for example menthylformiate, menthylacetate, menthylisobutyrate, menthyllactates, L-menthyl-L-lactate, L-menthyl-D-lactate, menthyl-(2-methoxy)acetate, menthyl-(2-methoxyethoxy-)acetate, menthylpyroglutamate), menthylcarbonates (for example menthylpropylenegly-colcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], as described in US 4,150,052**,** menthanecarboxylic acid-N-(4-cyanophenyl)amide or menthanecarboxylic acid-N-(4-cyanomethylphenyl)amide as described in WO 2005 049553 A1**,** methanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-l-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(l-isopropyl)cyclohexane-carboxamide [WS12], oxamates (preferably those described in EP 2033688 A2**).**

### L. Anti-microbial agents

Suitable anti-microbial agents are, in principle, all substances effective against Grampositive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### M. Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### N. Odour absorbers and antiperspirant active agents

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### O. Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol^{®} (4-acetyl-1-14-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### P. Carriers and Hydrotropes

**Preferred cosmetics carrier** materialsare solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a preparation according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Q. Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### R. Perfume oils and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, - isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### S. Dyes

Suitable dyes are any of the substances suitable and approved for cosmetic purposes as listed, for example, in the publication **"**Kosmetische Färbemittel" of the Farbstoff-kommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. Advantageous coloured pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

Preferred compositions according to the present inventions are selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the moisturizing and/or anti-ageing and/or wound healing promoting action thereof is more pronounced).

The formulations according to the invention are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular C₆-C₃₂ fatty acid C₂-C₃₀ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

The formulations according to the invention are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters)) or silicone oil, or a spray (e.g. pump spray or spray with propellant).

Auxiliary substances and additives can be included in quantities of 5 to 99 % b.w., preferably 10 to 80 % b.w., based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 % b.w., preferably 5 to 80 % b.w., based on the total weight of the preparation.

### PREPARATION EXAMPLES

### Example P1

### Preparation of cupuassu fatty acid dimethylamino propylamide

In a 1-I three-necked flask equipped with stirrer, reflux condenser and dropping funnel 580 (2 Mol) cupuassu butter ,their glycerides or methyl esters, and 1 g hypophosphoric acid were placed and heated to about 90 °C. Subsequently, 102 (1 Mol) dimethylamino propylamine were slowly added. The reaction takes place in a temperature graduation from 140°C up to 210°C, between four hours. Transamidation was continued until the amine value reached a value on the range of 140- 160 mg KOH/g.

The amido amide is washed to eliminate excess of amine, and looses glycerol.

### Example P2

### Preparation of a quaternized cupuassu fatty acid dimethylamino propylamide

In a 1-I three-necked flask equipped with stirrer, reflux condenser and dropping funnel 580 (2 Mol) cupuassu butter ,their glycerides or methyl esters, and 1 g hypophosphoric acid were placed and heated to about 90 °C. Subsequently, 102 (1 Mol) dimethylamino propylamine were slowly added. The reaction takes place in a temperature graduation from 140°C up to 210°C, between four hours. Transamidation was continued until the amine value reached a value on the range of 140- 160 mg KOH/g. 604 g (1 Mol) of the cupuassu fatty acid amidoamine thus obtained were solved at 50 °C in about 1 litre isopropyl alcohol and quaternized by adding drop wise 120 g (0.95 Mol) dimethyl sulfate. Once the addition was completed the mixture were stirred for another 2 h at about 80 °C and then cooled down to room temperature.

### Example P3

### Preparation of a 1:1 mixture of a quaternized and a non-quaternized cupuassu fatty acid dimethylamino propylamide

In a 1-I three-necked flask equipped with stirrer, reflux condenser and dropping funnel 580 (2 Mol) cupuassu butter ,their glycerides or methyl esters, and 1 g hypophosphoric acid were placed and heated to about 90 °C. Subsequently, 102 (1 Mol) dimethylamino propylamine were slowly added. The reaction takes place in a temperature graduation from 140°C up to 210°C, between four hours. Transamidation was continued until the amine value reached a value on the range of 140- 160 mg KOH/g. 604 g (1 Mol) of the cupuassu fatty acid amidoamine thus obtained were solved at 50 °C in about 1 litre isopropyl alcohol and quaternized by adding drop wise 60 g (0.48 Mol) dimethyl sulfate. Once the addition was completed the mixture were stirred for another 2 h at about 80 °C and then cooled down to room temperature.

### Comparative Example C1

### Preparation of tallow fatty acid dimethylamino propylamide

In a 1-I three-necked flask equipped with stirrer, reflux condenser and dropping funnel 540 (2 Mol) partly hydrogenated tallow fatty acid and 1 g hypophosphoric acid were placed and heated to about 90 °C. Subsequently, 102 (1 Mol) dimethylamino propylamine were added and temperature increased to about 200 °C. Transamidation was continued until the acid value reached a value of less than 7 mg KOH/g.

### Comparative Example C2

### Preparation of a quaternized tallow fatty acid dimethylamino propylamide

In a 1-I three-necked flask equipped with stirrer, reflux condenser and dropping funnel 540 (2 Mol) partly hydrogenated tallow fatty acid and 1 g hypophosphoric acid were placed and heated to about 90 °C. Subsequently, 102 (1 Mol) dimethylamino propylamine were added and temperature increased to about 200 °C. Transamidation was continued until the acid value reached a value of less than 7 mg KOH/g. 590 g (1 Mol) of the tallow fatty acid amidoamine thus obtained were solved at 50 °C in about 1 litre isopropyl alcohol and quaternized by adding drop wise 120 g (0.95 Mol) dimethyl sulfate. Once the addition was completed the mixture were stirred for another 2 h at about 80 °C and then cooled down to room temperature.

### Comparative Example C3

### Preparation of a 1:1 mixture of a quaternized and a non-quaternized tallow fatty acid dimethylamino propylamide

In a 1-I three-necked flask equipped with stirrer, reflux condenser and dropping funnel 540 (2 Mol) partly hydrogenated tallow fatty acid and 1 g hypophosphoric acid were placed and heated to about 90 °C. Subsequently, 102 (1 Mol) dimethylamino propylamine were added and temperature increased to about 200 °C. Transamidation was continued until the acid value reached a value of less than 7 mg KOH/g. 590 g (1 Mol) of the tallow fatty acid amidoamine thus obtained were solved at 50 °C in about 1 litre isopropyl alcohol and quaternized by adding drop wise 60 g (0.48 Mol) dimethyl sulfate. Once the addition was completed the mixture were stirred for another 2 h at about 80 °C and then cooled down to room temperature.

### APPLICATION EXAMPLES

**Table 1** exhibits compositions comprising the cupuassu fatty acid amides and/or their quaternization products 1 and 2 and the comparative products based on tallow fatty acid C1. The products were prepared by blending phase I, II and III at ambient temperatures. All test formulations contained the same concentrations of conditioning additive, 1,8% All amounts represent % by weight.

**Table 1**

| Leave-on conditioner formulations (placebo + three leave-on formulations) proposals. | | | | | |
|---|---|---|---|---|---|
| | **Formulation Number (FN)** | **Placebo Formulation A** | **1 Formulation B** | **C1 Formulation C** | **2 Formulation D** |
| **Phase** | **Component** | | | | |
| I | Cupuassu Amidopropyl Dimethylamine | 0.00 | 0.00 | 0.00 | 1.70 |
| | Dehyquart^{®} L80 Dicocoylethyl Hydroxyethylmonium Methosulfate(and)Propylene Glycol | 0.00 | 0.00 | 1.21 | 0.00 |
| | Blend 1 Cupuassu Amidopropyl Dimethylamine(and)Dicocoylethyl Hydroxyethylmonium Methosulfate(and)Propylene Glycol | 0.00 | 1.98 | 0.00 | |
| | Dehyquart^{®} S18 Stearamidopropyl Dimethylamine | 0.00 | 0.00 | 0.85 | 0.00 |
| | Latic acid | 0.00 | qs | qs | qs |
| | Water | Ad 100 | Ad 100 | Ad 100 | Ad 100 |
| | Glycerin | 2.00 | 2.00 | 2.00 | 2.00 |
| II | Lanette^{®} MY Cetearyl Alcohol | 4.00 | 4.00 | 4.00 | 4.00 |
| | Cutina^{®} MD Glyceryl Stearate | 1.00 | 1.00 | 1.00 | 1.00 |
| | Mineral Oil | 3.00 | 3.00 | 3.00 | 3.00 |
| III | Parfum | 0.50 | 0.50 | 0.50 | 0.50 |
| | DMDM Hydantoin | 0.20 | 0.20 | 0.20 | 0.20 |

In order, to verify the effect of the new composition of cupuassuamidopropyl dimethylamine, bleached hair were treated with leave-on formulations showing in table I. Quaternary ester was used as a comparison parameter, since it is largely used at hair care industry and it has good environmental friendly properties. The leave-on formulations (Table 1) were applied on each tress with gloved hands, uniformly distributed from root to tip ends. 0.2 g formulation/g hair was applied on each hair tress and triplicate hair tresses were used in each experiment.

### A. Hydro Retention Test

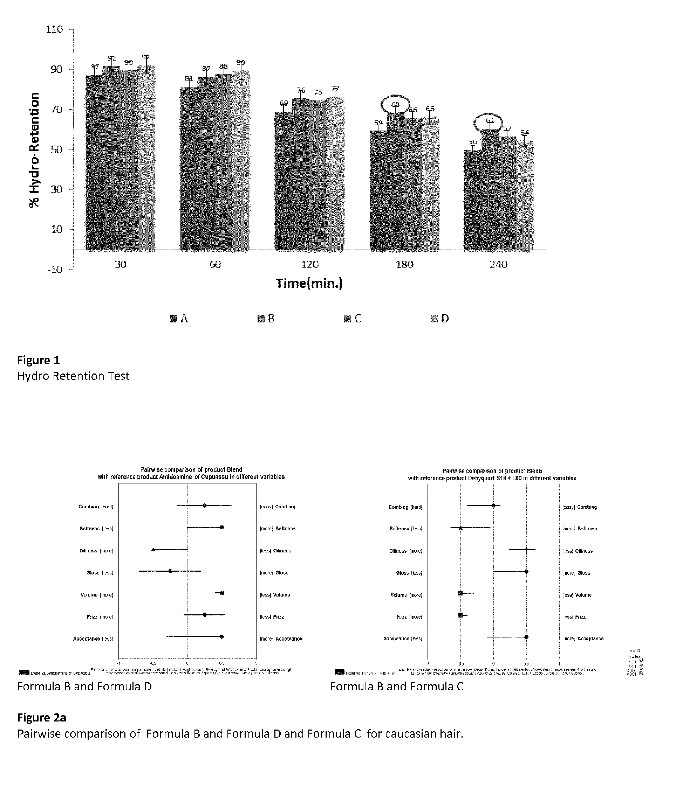

Each hair tress was weighed before the leave-on application. Subsequently after 24h conditioning, each hair tress was weighed. Subsequently each one was immerged in 100 mL of distilled water for 1.0 h; the excess water was eliminated using a towel and each tress was weighed again to calculate the adsorbed water. After the leave-on applications, each tress was weighed again (t=0h) and left drying in the conditioned room. After successive times (1.0; 2.0; 3.0 and 4.0h), the tresses were weighed. The subtraction of hair mass weighed at each successive time from the hair mass obtained at t=0h is the mass of water lost by the hair tresses. The results of the experiment are presented as percentage of hair hydroretention, which is calculated subtracting the values of mass of water lost from the values of adsorbed water for each hair tress and rationing this result by the values of adsorbed water. The results are compiled in **Figure 1****.**

The results for hydro retention clearly indicate that the products based on cupuassu fatty acid exhibit superior properties compared to the similar products from tallow (Dehyquart S18).

### B. Sensory Evaluation

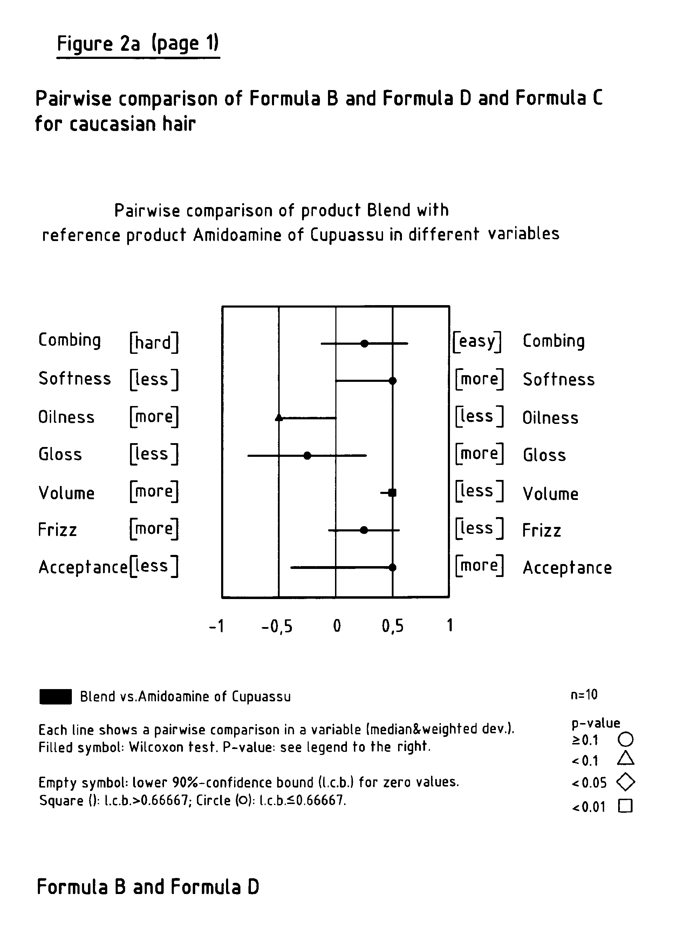

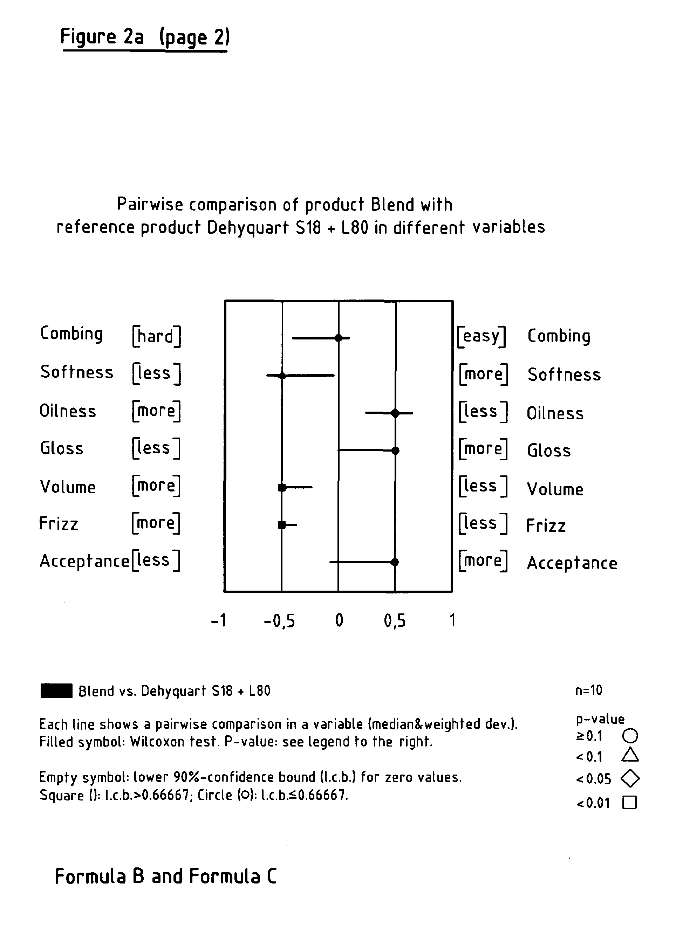

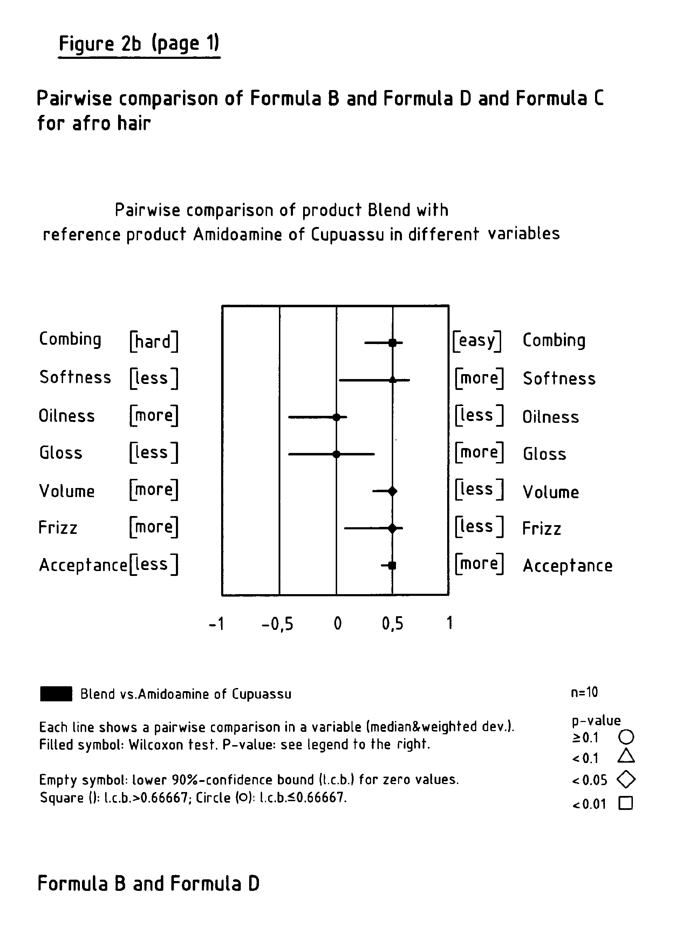

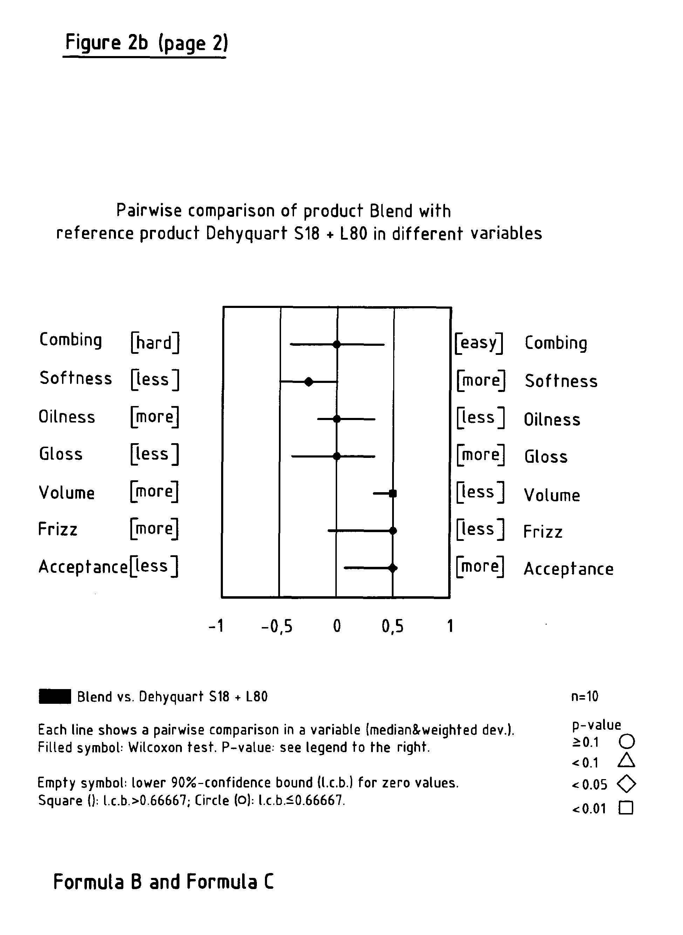

The standardized hair tresses were treated with the leave-on applications and were dried at room temperature (25°C) and 60% RH. Ten volunteers evaluated smoothness, combing, oiliness, gloss, volume, frizz and final acceptance. The results were evaluated by a pairwise comparison of sensoric data using a statistical program (Sensorics - Wilcoxon test). Were performed tests to caucasin hair and afro hair? The results are compiled in **Figures 2a** and **2b****.** The results indicated in **Figures 3a and 3b** illustrate the results obtained for each pairwise of the test formulations. The results indicate that Formula D comprising the cupuassu fatty acid amidoamine and Formula C comprising the similar products from tallow had lower percentage of acceptance then Formula D, which represent the mixture of cupuassu fatty acid amidoamine and a quaternary compound.

### C. Cold process

In order to verify feasibility to use this mixture of cupuassu fatty acid amidoamine and a quaternary compound were performed an test using cold processable formulation since it is an tendency to reduce the use of energy in order to reach an more sustainable process. **Table 2** exhibits products 3 and 4 which were prepared by blending phase I, II and III at ambient temperatures. Different amounts of this blend were used to investigate to the behaviour of the formulation using different concentrations of conditioning additive. The **Figures 3** show the aspect of the formulations obtained, that are creamy and rich. The process was very easy to handle and without heat any component.

**Table 2**

| Leave-on conditioner formulations (cold processable formulation) proposals. | | | |
|---|---|---|---|
| **Phase** | **Formulation Number (FN)** | **Placebo Formulation A** | **1 Formulation B** |
| I | Comedia-Triple C | | |
| | *Polyquaternium-37 (and) Dicaprylyl Carbonate (and) Lauryl Glucoside* | 2.00 | 2.70 |
| | IPP^{®} *Isopropyl Palmitate* | 4.00 | 4.00 |
| | Water | Ad 100 | Ad 100 |
| II | Luvigel^{®} Star | | |
| | *Polyurethane-39* | 3 | 3 |
| | Blend 1 | | |
| | *Cupuassu Amidopropyl Dimethylamine(and)Dicocoylethyl Hydroxyethylmonium Methosulfate(and)Propylene Glycol* | **1.21** | **1.98** |
| III | *DMDM Hydantoin* | 0.20 | 0.20 |
| | *Parfum* | 0.50 | 0.50 |

### D. Viscosity evaluation

To evaluate the impact of the Blend 1 (Cupuassu Amidopropyl Dimethylamine (and) Dicocoylethyl Hydroxyethylmonium Methosulfate(and)Propylene Glycol) on the final viscosity of the leave-on formulations 3 and 4, their viscosities were evaluated at Brookfield RV DV II at room temperature (25°C). The results are shown on **Table 3.**

**Table 3**

| | | |
|---|---|---|
| Viscosity evaluation (cold processable formulation) | | |
| **Viscosity ty (Brookfield RVT-Helipath/Spindle C/25ºC)** | **3** | **4** |
| | 9500cps | 12100cps |

The results indicated Table 3 show that this blend doesn't have any negative impact of viscosity of leave-on formulations, even on different concentrations.

## Claims

1. A cupuassu fatty acid amidoamine according to formula (I) 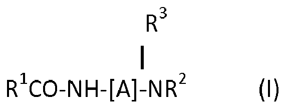 in which
R¹CO stands for a mixture of acyl radicals having 12 to 22 carbon atoms;
R² and R³ independently from each other represent hydrogen or alkyl radicals having 1 to 6 carbon atoms;
A stands for a linear or branched alkylene radical having 1 to 6 carbon atoms on condition that the mixture of acyl radicals represent the fatty acid spectrum of cupuassu butter *(Theobroma grandiflorum).*

2. A cupuassu quaternary fatty acid amidoamine according to formula (II) 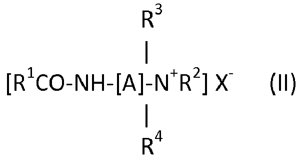 in which
R¹CO stands for a mixture of acyl radicals having 12 to 22 carbon atoms;
R², R³ and R⁴ independently from each other represent hydrogen or alkyl radicals having 1 to 6 carbon atoms;
A stands for a linear or branched alkylene radical having 1 to 6 carbon atoms; and X represents a halogenide, sulfate or carbonate anion;
on condition that the mixture of acyl radicals represent the fatty acid spectrum of cupuassu butter *(Theobroma grandiflorum).*

3. A mixture of a cupuassu fatty acid amidoamines of formula (I) and a quaternary cupuassu fatty acid amidoamine of formula (II) in a weight ratio of about 10:90 to about 90:10.

4. The mixture of Claim 3, obtainable in that cupuassu butter is subjected to transamidation and the amidoamines thus obtained are partly subjected to quaternization, whereby the amount of quaternization agent is adjusted so that the desired ratio of non-quaternized and quaternized amidoamines is automatically achieved.

5. The mixture of Claim 3, comprising also phytochemicals.

6. A neutralized cupuassu fatty acid amidoamine according to formula (Ib) 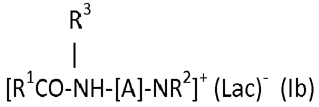 in which
R¹CO stands for a mixture of acyl radicals having 12 to 22 carbon atoms;
R² and R³ independently from each other represent hydrogen or alkyl radicals having 1 to 6 carbon atoms;
A stands for a linear or branched alkylene radical having 1 to 6 carbon atoms, and Lac stands for a lactate anion,
on condition that the mixture of acyl radicals represent the fatty acid spectrum of cupuassu butter *(Theobroma grandiflorum).*

7. A process for making cupuassu fatty acid amidoamines and/or quaternized cupuassu fatty acid amidoamines in that
(a) the oil from *Theobroma grandiflorum* is subjected to transamidation with diamine of formula (III)
NH₂-[A]-NR²R³ (III)
wherein A, R² and R³ have the same meanings as defined above, and optionally
(b) the cupuassu fatty acid amidoamine thus obtained is reacted with an alkyl halogenide, a dialkyl sulfate or a dialkyl carbonate to provide the quaternized cupuassu fatty acid amidoamine.

8. The process of Claim 6, wherein the diamine is selected from the group consisting of etehylenediamine, propylenediamine, butylenediamine, dimethylaminoethylamine, dimethylaminopropylamine, dimethylaminobutylamine, dimethylaminopentylamine, dimethylaminohexylamine, diethylamineethylamine, diethylaminopropylamine, diethylaminobutylamine, diethylaminopentylamine, diethylaminohexylamine, methylethylaminoethylamine, methylethylaminopropylamine, methylethylaminobutylamine, methylethylaminopentylamine, methylethylaminohexylamine and their mixtures.

9. The process of Claim 6, wherein the fatty acid amidoamine intermediate and the quaternization agent are reacted in a molar ratio so that the resulting mixture comprises the non-quaternized and the quaternized fatty acid amidoamine in any desired weight ratio of about 10:90 to 90:10.

10. A personal care composition comprising
(a) a cupuassu fatty acid amidoamines of Claim 1 or Claim 6, and
(b1) a quaternized cupuassu fatty acid amidoamines of Claim 2 and/or
(b2) a quaternary conditioning agent.

11. A cosmetic or pharmaceutical composition comprising
(a) a cupuassu fatty acid amidoamines of Claim 1 or Claim 6, and
(b1) a quaternized cupuassu fatty acid amidoamines of Claim 2 and/or
(b2) a quaternary conditioning agent.

12. The composition of Claim 10 or Claim 11 comprising the cupuassu fatty acid amidoamines and/or the quaternized fatty acid amidoamines in a total amount of from about 0.5 to about 25 % by weight - calculated on the final compositions.

13. A method for wettability, for providing moisture and for reducing electrostatic charge of human hair, the improvement comprising applying
(i) the cupuassu fatty acid amidoamines of Claim 1 or Claim 6,
(ii) the quaternized cupuassu fatty acid amidoamines of Claim 2,
(iii) the mixtures of Claim 3 or
(iv) the compositions of Claim 10 or 11
to hair.

14. A method for improving the moisture status of human skin, the improvement comprising applying
(i) the cupuassu fatty acid amidoamines of Claim 1 or Claim 6,
(ii) the quaternized cupuassu fatty acid amidoamines of Claim 2,
(iii) the mixtures of Claim 3 or
(iv) the compositions of Claim 10 or 11
to skin.

15. The use of cupuassu fatty acid amidoamines of Claim 1 or 6, of quaternized cupuassu fatty acid amidoamines of Claim 2 or their mixtures of Claim 3 for making personal care, cosmetic or pharmaceutical compositions.
